# EUROPÄISCHE PATENTANMELDUNG

(11) **EP 0 982 293 A1**
(43) Veröffentlichungstag der Anmeldung: **01.03.2000**
(21) Anmeldenummer: 99115925.2
(22) Anmeldetag: 13.08.1999
(51) Int. Cl.: C07C 249/02, C07C 209/60

(54) **Verfahren zur Herstellung von Iminen und Enaminen und gegebenenfalls deren Weiterhydrierung zu Aminen**

(30) Priorität: 14.08.1998 DE 19836814
(71) Anmelder: BASF AKTIENGESELLSCHAFT, 67056 Ludwigshafen (DE)
(72) Erfinder: Breuer, Klaus, Dr., 67122 Altrip (DE); Teles, Joaquim Henrique, Dr., 67122 Altrip (DE); Rieber, Norbert, Dr., 68259 Mannheim (DE); Demuth, Dirk, Dr., 68161 Mannheim (DE); Hibst, Hartmut, Prof. Dr., 69198 Schriesheim (DE)

(57) **Zusammenfassung**

Verfahren zur Herstellung von Iminen und/oder Enaminen durch Anlagerung von Ammoniak, primären Aminen oder sekundären Aminen an Acetylene und/oder Allene, bei denen man die Umsetzung in der Gasphase bei erhöhter Temperatur in Gegenwart von einem amorphes und/oder kristallines Zink- und/oder Cadmiumsilikat enthaltenden Heterogenkatalysator durchführt.

## Beschreibung

Die Erfindung betrifft ein Verfahren zur Herstellung von Iminen und Enaminen durch Anlagerung von Ammoniak, primären Aminen oder sekundären Aminen an Acetylene oder Allene in der Gasphase an heterogenen, Zink- und/oder Cadmiumsilikat enthaltenden Katalysatoren und gegebenenfalls Hydrierung der erhaltenen Imine oder Enamine zu Aminen.

Es ist bekannt, Umsetzungen von Alkinen mit Aminen in der Flüssigphase in gefluteten Reaktoren durchzuführen. Beispiele für Katalysatoren sind Kupfersalze (BE 637.033) sowie eine Vielzahl von Basensystemen wie KOH (SU 464 584) bis hin zu den Alkalimetallsalzen der eingesetzten Amine (US 3,337,577). Außerdem sind Quecksilbersalze enthaltende Systeme (HgOAc₂ oder HgOAc₂ + Base) bekannt, die in kondensierter Phase die Addition von Aminen an Alkine katalysieren (Barluenga et al., Chem. Commun. 1985, 1375; Barluenga et al., Tetrahedron Lett. 1995, 36, 6551-6554). Darüberhinaus wurden auch Au(III)-Katalysatoren für die intramolekulare Addition von Aminofunktionen an Dreifachbindugen beschrieben (K. Utimoto et al., Synthesis 1991, 975-978).

In der Flüssigphase ist die Vinylierung von Aminen auch schon an Zn- oder Cd-Katalysatoren beschrieben worden (GB 887 365, US 3,480,627, US 589,537 und US 3,179,661). Nachteilig an den bekannten Verfahren in der Flüssigphase sind auftretende Sicherheitsprobleme durch die Verwendung von Alkinen unter Druck.

Die Reaktion von Aminen mit Alkinen in der Gasphase ist bei Normaldruck und 150 bis 350°C für die Umsetzung von Pyrrol mit Acetylen an basischen Katalysatoren (MgO/CaO/KOH) bekannt (US 3,047,583). Eine weitere Gasphasenvinylierung von Aminen ist in SU 268 431 beschrieben. Diese verläuft bei 300 bis 350°C an Alkalimetall (hydr)oxid-Katalysatoren.

Ein weiteres Verfahren zur Gasphasenvinylierung von Aminen ist von R.S. Neale et al. im J. Catal. 27 (1972) 432-441, beschrieben. Hierbei werden Y-Zeolithe als Katalysatoren verwendet, in denen Natriumionen durch Zink(II)-Ionen ausgetauscht wurden.

Die genannten Gasphasenverfahren haben eine Reihe von Nachteilen. So verlaufen sie mit unzureichenden Selektivitäten, Raum-Zeit-Ausbeuten und Katalysatorstandzeiten und sind nicht universell für alle Amine einsetzbar.

Es bestand daher die Aufgabe, ein Verfahren vorzuschlagen, das vor allem höhere Raum-Zeit-Ausbeuten und längere Katalysatorstandzeiten erlaubt, bei Normaldruck durchgeführt werden kann und universell, d.h. für die Umsetzung der verschiedensten Amine anwendbar ist.

Diese Aufgabe wurde erfindungsgemäß gelöst mit einem Verfahren zur Herstellung von Iminen und/oder Enaminen durch Anlagerung von Ammoniak, primären Aminen oder sekundären Aminen an Acetylene und/oder Allene, wobei man die Umsetzung in der Gasphase bei erhöhter Temperatur in Gegenwart von einem amorphes und/oder kristallines Zink- und/oder Cadmiumsilikat enthaltenden Heterogenkatalysator durchführt und insbesondere mit einem Verfahren zur Herstellung von Iminen der Formel I und/oder Enaminen der Formel II 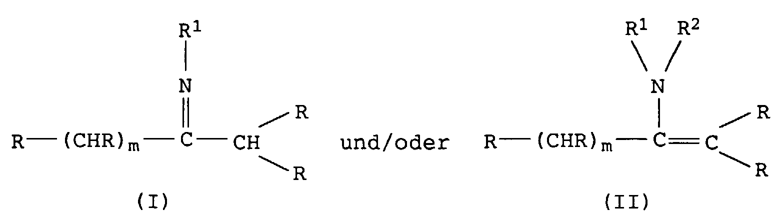 in denen die Reste R unabhängig voneinander für Wasserstoff oder aliphatische, cycloaliphatische, araliphatische, aromatische oder heterocyclische Reste stehen und R¹ und R² unabhängig voneinander Wasserstoff, aliphatische, cycloaliphatische, araliphatische, aromatische oder heterocyclische Reste bedeuten und R¹ und R² gemeinsam einen heterocyclischen Ring bilden können und m für eine ganze Zahl von 0 bis 8 steht, durch Umsetzung von Ammoniak, primären Aminen oder sekundären Aminen der Formeln III bzw. IV

R¹NH₂ (III)

bzw. R¹R²NH (IV)

mit Acetylenen der Formel V bzw. Allenen der Formel VI 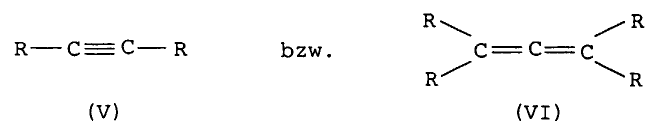 wobei R¹, R² und R die oben genannten Bedeutungen haben, das dadurch gekennzeichnet ist, dass man die Umsetzung in der Gasphase in Gegenwart von einem amorphes und/oder kristallines Zink- und/oder Cadmiumsilikat enthaltenden Heterogenkatalysator bei Drücken von 0,1 bis 50 bar und Temperaturen von 100 bis 370°C durchführt.

Bei der erfindungsgemäßen Umsetzung erhält man Imine bzw. Enamine, wie dies mit dem folgenden Formelschema für die Umsetzung von Ethylamin bzw. Diethylamin mit Propin exemplifiziert ist.

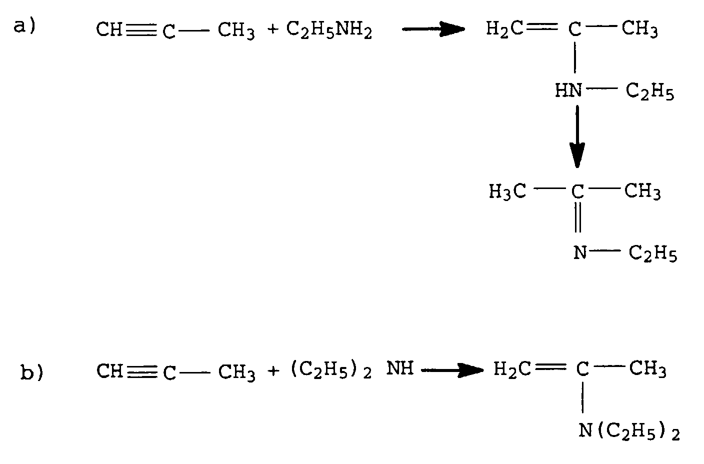

Dabei findet die Anlagerung überwiegend am Kohlenstoff 2 des Propins, also zur Isopropenylverbindung statt und in geringerem Maße am Kohlenstoff 1, so daß zum Teil auch das n-Isomere der Formel 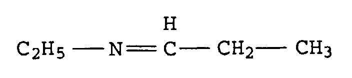 gebildet wird.

Als Edukte kommen neben Ammoniak an sich beliebige primäre oder sekundäre Amine in Betracht, doch sind Alkyl- und Cycloalkylamine mit 1 bis 12, insbesondere 1 bis 6 C-Atomen sowie Iminogruppen enthaltende Heterocylen mit 2 bis 7, insbesondere 3 bis 5 C-Atomen, bevorzugt. Die Amine können gegebenenfalls weitere Substituenten, die unter den Reaktionsbedingungen nicht mit Acetylenen oder Allenen reagieren, tragen. Im einzelnen seien genannt:
Methylamin, Dimethylamin, Ethylamin, Diethylamin, Methylethylamin, n-Propylamin, Di-n-propylamin, Isopropylamin, Diisopropylamin, N-Methyl-isopropylamin, N-Ethyl-isopropylamin, N-Ethyl-n-propylamin, n-Butylamin, Isobutylamin, sek.-Butylamin, tert.-Butylamin, (2-Ethylhexyl)amin, n-Pentylamin, 2-Methyl-butylamin, Ethanolamin, Ethylendiamin, 1-Methoxy-2-amino-propan, Cyclohexylamin, Anilin, Benzylamin, N-Methyl-benzylamin, 1-Phenyl-ethylamin und 2-Phenyl-ethylamin sowie Heterocyclen, wie Pyrrolidin, Piperidin, Piperazin, N-Methyl-piperazin, Morpholin, Pyrrol, Imidazol und Pyrazol.

Als Acetylene und Allene kommen beliebige, die Acetylen- oder Allengruppierung enthaltende Verbindungen in Betracht.

Jedoch verwendet man vor allem die technisch leicht zugänglichen Acetylene und/oder Allene mit 2 bzw. 3 bis 8 C-Atomen, und insbesondere Acetylen, Methylacetylen oder Allen oder deren Gemische, wie sie beispielsweise aus einem C₃-Strom von einem Steamcracker isoliert werden können.

Als Zink- und/oder Cadmiumsilikat enthaltende Katalysatoren kommen bevorzugt Zink- oder Cadmiumsilikate enthaltende Katalysatoren in Betracht, die aus der im folgenden erläuterten Gruppe (a), (b) und (c) ausgewählt sind:
(a) röntgenamorphes Zinksilikat oder Cadmiumsilikat, hergestellt durch Imprägnierung eines Kieselsäureträgers mit einem Zink- bzw. Cadmiumsalz,
(b) kristallines Zinksilikat mit im wesentlichen der Zusammensetzung und Struktur des Hemimorphits der Formel Zn₄Si₂O₇(OH)₂·H₂O, wobei das Zink in einem bis zu 25%igen Unter- oder Überschuß, bezogen auf die stöchiometrische Zusammensetzung vorliegen kann und/oder
(c) im wesentlichen röntgenamorphes Zinksilikat, hergestellt durch Ausfällen in wäßriger Lösung aus einer löslichen Silicium- und Zinkverbindung, der Formel VII

   ZnₐSi_{c}O_{a+2c-0,5e}(OH)ₑ · f H₂O VII,

   in der e die Werte 0 bis zur Summe aus 2a+4c bedeutet, das Verhältnis a/c 1 bis 3,5 und f/a 0 bis 200 beträgt.

- Zu (a):: Röntgenamorphe Zinksilikat- oder Cadmiumsilikat-Katalysatoren werden z.B. durch Beladen von amorpher Kieselsäure mit einem Zinksalz bzw. Cadmiumsalz und Formierung des Katalysators durch eine thermische Behandlung erhalten.
Der SiO₂-Träger ist zumindest überwiegend amorph, hat eine BET-Oberfläche zwischen 10 und 1500 m²/g, vorzugsweise 100 bis 500 m²/g, eine Wasseraufnahmekapazität von 0,1 bis 2 ml/g, vorzugsweise von 0,7 bis 1,3 ml/g und kann als Pulver oder als fertiger Formkörper eingesetzt werden. Der Träger kann auch vor der Imprägnierung kalziniert werden. Bevorzugt wird der Träger aber nicht kalziniert.
Als Zink- bzw. Cadmiumverbindung verwendet man eine in einem geeigneten Lösungsmittel lösliche Verbindung. Bevorzugt werden Zink(II)-Salze verwendet, die in Wasser oder wäßrigem Ammoniak oder Alkoholen, bevorzugt niederen Alkoholen, löslich sind und deren Zersetzungstemperatur unterhalb 400°C bis 500°C liegt.
Besonders bevorzugt wird für die Imprägnierung eine ammoniakalische Zink(II)acetat-Lösung verwendet. In manchen Fällen hat es sich als vorteilhaft erwiesen, die Beladung mit Zink in mehreren aufeinander folgenden Tränkschritten vorzunehmen.
Wenn der Träger als Pulver eingesetzt wird, kann der Katalysator durch Formgebung (z.B. durch Mischen, Kneten und Verstrangen oder Tablettieren) in die gewünschte Form gebracht werden.
Zur Erhöhung des Porenvolumens können bei der Formgebung auch Porenbildner zugesetzt werden (z.B. Superabsorber wie Lutexal® P (Firma BASF, Ludwigshafen) oder Walocel® (Methylcellulose/Kunstharz-Kombination, Firma Wolff, Walsrode)).
Alternativ kann auch ein anderer Träger, z.B. Al₂O₃, mit einer Siliciumoxid-Vorläuferverbindung (z.B. Si(OR)₄) und mit einem zinksalz oder Cadmiumsalz imprägniert werden.
Die Zink- bzw. Cadmiumbeladung kann in weiten Grenzen variieren. Typische Werte für einen unkalzinierten Präkatalysator, der durch Tränkung eines SiO₂-Trägers mit einem Zinksalz bzw. Cadmiumsalz hergestellt wurde, liegen z.B. zwischen 1 und 60 Gew.-% Zn oder Cd, vorzugsweise zwischen 7 und 30 Gew.-%, insbesondere zwischen 10 und 25 Gew.-% (jeweils berechnet als ZnO oder CdO). Der Präkatalysator kann außerdem mit anderen Elementen dotiert werden, bevorzugt mit Alkali-, Erdalkali- oder Übergangsmetallen. Ferner kann die katalytisch wirksame Komponente mit bis zu 80, vorzugsweise bis zu 50 und insbesondere bis zu 20 Molprozent noch mit weiteren Metallen dotiert sein, ausgewählt aus der Gruppe (A) bestehend aus Beryllium, Magnesium, Calcium, Strontium, Barium, Mangan, Eisen, Kobalt, Nickel und Kupfer, und der Gruppe (B), bestehend aus Titan, Zirkon, Hafnium, Germanium, Zinn und Blei, wobei die Elemente der Gruppe (A) teilweise Zink, bzw. Cadmium und die Elemente der Gruppe (B) teilweise Silicium ersetzen.
Der Präkatalysator kann dann bei einer Temperatur von maximal 600°C, vorzugsweise zwischen 80 und 400°C, an Luft oder unter Inertgas kalziniert werden. Besonders bevorzugt ist die Kalzinierung zwischen 120 und 325°C an Luft.
Nach der Herstellung des im allgemeinen katalytisch noch inaktiven oder weniger aktiven Präkatalysators, durch Aufbringung einer Zink- und/oder Cadmiumverbindung auf einen Siliciumoxid-Träger wird im allgemeinen eine Formierung durchgeführt, bei der sich - vor allem auf der Oberfläche des Katalysators - die eigentliche Aktivphase ausbildet. Diese Festkörperreaktion wird durch die Anwesenheit von Wasser, Alkoholen, bevorzugt niederen Alkoholen, Carbonsäuren, bevorzugt niederen Carbonsäuren, Ammoniak oder Ammen, gefördert und wird deshalb zweckmäßigerweise durch Erhitzen des Präkatalysators bei einer Temperatur zwischen 50 und 400°C in einer wäßrigen, Alkohol und/oder Amin enthaltenden Atmosphäre durchgeführt. Bevorzugt führt man die Reaktion zwischen 100 und 350°C in einem wäßrigen, Methanol und/oder Ethylamin enthaltenden Gasgemisch aus. Besonders bevorzugt ist die Durchführung der Reaktion zwischen 120 und 300°C mit einem aminhaltigen Gasgemisch direkt in dem Reaktor, in dem später die Umsetzung mit dem Alkin oder Allen stattfinden soll. Als Amine verwendet man dabei mit Vorteil die gleichen Amine, die anschließend umgesetzt werden sollen.
Auf die gleiche Weise können auch die entsprechenden Quecksilbersilikate hergestellt werden, die jedoch technisch und ökologisch weniger geeignet sind.
- Zu (b):: Hemimorphit als Katalysator
Hemimorphit ist ein Zinksilikat der Formel Zn₄Si₂O₇(OH)₂ · H₂O. Für die erfindungsgemäße Umsetzung sind jedoch nicht nur reiner Hemimorphit, sondern allgemein heterogene Katalysatoren geeignet, die zumindest überwiegend als Aktivkomponente Zinksilikat mit der Struktur des Hemimorphits der Formel Zn₄Si₂O₇(OH)_{2-2y}O_{y} · xH₂O enthalten, wobei x und y für die Werte 0 bis 1 stehen. Die Herstellung von Hemimorphit ist aus der Literatur bekannt. Sie kann unter Normalbedingungen oder hydrothermalen Bedingungen erfolgen. Bevorzugt wird ein Hemimorphit verwendet wie er gemäß den Angaben in der deutschen Patentanmeldung Nr. 19726670.3 erhalten wird.
- Zu (c):: Röntgenamorpher Zinksilikat-Katalysator
Gemäß den Angaben in der deutschen Patentanmeldung Nr. 19726670.3 kann mit kürzeren Umsetzungszeiten, als für die Vorstufe zur Herstellung eines kristallinen Hemimorphits erforderlich ist, ein röntgenamorphes Produkt mit verbesserten katalytischen Eigenschaften erhalten werden.
Dazu wird z.B. eine wäßrige Suspension oder Lösung eines Alkali- oder Erdalkalisilikats mit einer wäßrigen Lösung eines Zinksalzes bei
a) Temperaturen von 20°C, bevorzugt 50°C bis zum Siedepunkt der sich ergebenden wäßrigen Suspension bei
b) einem pH-Wert von 4 bis 9,5, vorzugsweise bei einem pH-Wert in der Nähe des Neutralpunktes,
c) und solchen Mengenverhältnissen von Alkalisilikat und Zinksalz umgesetzt, daß die Bedingungen der Formel VII erfüllt werden, und
d) unter Einhaltung einer solchen Verweilzeit umgesetzt, daß noch nicht in erheblichem Maße Kristallisation des gebildeten Zinksilikats eintritt.
Das so erhältliche, im wesentlichen röntgenamorphe Zinksilikat enthält Zn²⁺-, Si⁴⁺ und O²-Ionen; darüber hinaus kann die Verbindung OH-Ionen und Hydratwasser enthalten. Das Zn/Si-Verhältnis beträgt 0,3 bis 5, vorzugsweise 1 bis 2,7, insbesondere 2 bis 2,3.

Auch der erfindungsgemäß zu verwendende amorphe Zinksilikat-Fällungskatalysator kann mit bis zu 80, vorzugsweise bis zu 50, und insbesondere bis zu 20 Molprozent, noch mit weiteren Metallen dotiert sein ausgewählt aus der Gruppe (A) bestehend aus Beryllium, Magnesium, Calcium, Strontium, Barium, Mangan, Eisen, Kobalt, Nickel, Kupfer, Cadmium und Quecksilber und der Gruppe (B), bestehend aus Titan, Zirkonium, Hafnium, Germanium, Zinn und Blei, wobei die Elemente der Gruppe (A) teilweise Zink und die Elemente der Gruppe (B) teilweise Silicium in der Hemimorphit-Struktur ersetzen.

Die Umsetzung von Ammoniak, der primären und sekundären Amine mit den Acetylenen bzw. Allenen erfolgt in Gegenwart des heterogen vorliegenden Zink- und/oder Cadmiumsilikat enthaltenden Katalysators in der Gasphase entweder über einem Festbett oder in einem Wirbelbett bei Temperaturen von 100 bis 370°C, vorzugsweise 150 bis 320°C und insbesondere 180 bis 280°C und Drücken von 0,1 bis 50 bar, vorzugsweise 0,8 bis 20 bar und besonders bevorzugt 0,9 bis 10 bar (alle Drücke bezogen auf die Summe der Partialdrücke der Edukte), insbesondere bei Normaldruck.

Gegebenenfalls kann das Reaktionsgemisch aus Gründen der Betriebssicherheit und der besseren Wärmeabfuhr mit Inertgasen, wie Stickstoff, Argon, niedermolekularen Alkanen oder Olefinen verdünnt werden.

Die Amine werden mit den Acetylenen bzw. Allenen im allgemeinen im Molverhältnis von Amin zu Acetylen bzw. Allen von 0,01 bis 100, vorzugsweise 0,3 bis 5, insbesondere 0,7 bis 2,0, umgesetzt. Nicht umgesetztes Amin kann aus dem Reaktionsgemisch isoliert und in die Reaktion zurückgeführt werden. Nach einer speziellen Ausführungsform der Erfindung werden die erhaltenen Imine bzw. Enamine in an sich bekannter Weise katalytisch hydriert. Dadurch erhält man aus den ursprünglich eingesetzten primären Aminen sekundäre Amine und aus den sekundären Aminen tertiäre Amine.

Für die katalytische Hydrierung kann man das Reaktionsgemisch unmittelbar oder die daraus isolierten Imine oder Enamine einsetzen.

Die Hydrierung erfolgt unter literaturbekannten Bedingungen, wie sie z.B. in Houben-Weyl, Methoden der organischen Chemie, Org. Strickstoffverb. Teil 2, Bd. E/6d, 4. Aufl., 1992, Seiten 899-923, und JACS 66 (1944), Seite 82, beschrieben sind. Zweckmäßig wählt man als Reaktionsbedingungen Temperaturen von 0 bis 200°C, vorzugsweise von 20 bis 150°C und Drücke von 0,3 bis 300 bar (Wasserstoffpartialdruck).

Als Hydrierkatalysatoren seien beispielsweise Pd/C, Pd(OH)₂/C, Pt/C und Nickel genannt.

Das erfindungsgemäße Verfahren erlaubt in einfacher Weise die Herstellung von Iminen bzw. Enaminen oder über deren Hydrierung die Alkylierung von primären und sekundären Aminen.

### Beispiele 1 und 2

20 g/h Ethylamin wurden in einem Vorverdampfer verdampft und dann mit 10 l/h Propin bei Normaldruck in einen Rohrreaktor bei 210 bzw. 230°C über 100 ml eines amorphen Zinksilikats (Herstellung ist beschrieben in Beispiel 1 der deutschen Patentanmeldung Nr. 19726666.5) umgesetzt. Der Reaktoraustrag wurde kondensiert und gaschromatographisch untersucht. Die Umsätze, bezogen auf Ethylamin, und die Selektivitäten für N-Ethyl-propenylamin, bezogen auf umgesetztes Ethylamin, sind in der folgenden Tabelle 1 aufgelistet, ebenso jeweils das erzielte Verhältnis von N-Ethyl-2-propenylamin zu N-Ethyl-1-propenylamin (iso/n-Verhältnis).

**Tabelle 1**

| Beispiel | Temperatur [°C] | Umsatz [%] | Selektivität [%] | iso/n-Verhältnis |
|---|---|---|---|---|
| 1 | 210 | 34 | 93 | 8 |
| 2 | 230 | 49 | 92 | 10 |

### Beispiel 3

26,6 g/h Isopropylamin wurden in einem Vorverdampfer verdampft und dann mit 10 l/h Propin bei Normaldruck in einen Rohrreaktor bei 230°C über 100 ml des für die Beispiele 1 und 2 angegebenen amorphen Zinksilikats umgesetzt. Der Reaktoraustrag wurde kondensiert und gaschromatographisch untersucht. Der Umsatz, bezogen auf Isopropylamin, betrug 23%. Die Selektivität für N-Isopropylpropenylamin, bezogen auf umgesetztes Isopropylamin, lag bei 91 %. Das Verhältnis von N-Isopropyl-2-propenylamin zu N-Isopropyl-1-propenylamin betrug 4.

### Beispiel 4

33 g/h Diethylamin wurden in einem Vorverdampfer verdampft und dann mit 10 l/h Propin bei Normaldruck in einen Rohrreaktor bei 230°C über 100 ml des für Beispiele 1 und 2 beschriebenen amorphen Zinksilikats umgesetzt. Der Reaktoraustrag wurde kondensiert und gaschromatographisch untersucht. Der Umsatz, bezogen auf Diethylamin, betrug 26%. Die Selektivität für N,N-Diethyl-propenylamin, bezogen auf umgesetztes Diethylamin, lag bei 86%. Das Verhältnis von N,N-Diethyl-2-propenylamin zu N,N-Diethyl-1-propenylamin betrug 7.

### Beispiel 5

19,6 g/h N-Ethyl-N-isopropylamin wurden in einem Vorverdampfer verdampft und dann mit 5 l/h Propin und 5 l/h Stickstoff bei Normaldruck in einen Rohrreaktor bei 230°C über 100 ml des für Beispiele 1 und 2 beschriebenen amorphen Zinksilikats umgesetzt. Der Reaktoraustrag wurde kondensiert und gaschromatographisch untersucht. Der Umsatz, bezogen auf N-Ethyl-N-isopropylamin, betrug 23%. Die Selektivität für N-Ethyl-N-isopropyl-propenylamin, bezogen auf umgesetztes N-Ethyl-N-isopropylamin, lag bei 91 %. Das Verhältnis von N-Ethyl-N-isopropyl-2-propenylamin zu N-Ethyl-N-isopropyl-1-propenylamin betrug 6.

### Beispiel 6

30 g/h Pyrrol wurden in einem Vorverdampfer verdampft und dann mit 10 l/h Propin bei Normaldruck in einen Rohrreaktor bei 230°C über 100 ml eines amorphen Zinksilikat (wie in Beispiel 1/2) umgesetzt. Der Reaktoraustrag wurde kondensiert und gaschromatographisch untersucht. Der Umsatz, bezogen auf Pyrrol, betrug 36 %. Die Selektivität für N-Propenyl-pyrrol, bezogen auf umgesetztes Pyrrol, lag bei 61 %. Das Verhältnis N-(2-Propenyl)-pyrrol zu N-(1-Propenyl)-pyrrol betrug 6.

### Beispiel 7 und 8

20 g/h Pyrrolidin wurden in einem Vorverdampfer verdampft und dann mit 10 l/h Propin bei Normaldruck in einen Rohrreaktor bei 210 bzw. 230°C über 40 ml des für Beispiele 1 und 2 beschriebenen amorphen Zinksilikats umgesetzt. Der Reaktoraustrag wurde kondensiert und gaschromatographisch untersucht. Die Umsätze, bezogen auf Pyrrolidin, und die Selektivitäten für für N-Propenylpyrrolidin, bezogen auf umgesetztes Pyrrolidin, sind in der folgenden Tabelle 2 aufgelistet, ebenso die Verhältnisse von N-(2-Propenyl)-pyrrolidin zu N-(1-Propenyl)-pyrrolidin.

**Tabelle 2**

| Beispiel | Temperatur [°C] | Umsatz [%] | Selektivität [%] | iso/n-Verhältnis |
|---|---|---|---|---|
| 7 | 200 | 20 | 25 | 6 |
| 8 | 230 | 61 | 23 | 6 |

### Beispiel 9

20 g/h Pyrrolidin wurden in einem Vorverdampfer verdampft und dann mit 10 l/h Propin bei Normaldruck in einen Rohrreaktor bei 230°C über 40 ml eines Hemimorphit-Katalysators (die Herstellung ist beschrieben in Beispiel 3 der deutschen Patentanmeldung Nr. 19726670.3) umgesetzt. Der Reaktoraustrag wurde kondensiert und gaschromatographisch untersucht. Der Umsatz, bezogen auf Pyrrolidin, betrug 69%. Die Selektivität für N-Propenyl-pyrrolidin, bezogen auf umgesetztes Pyrrolidin, lag bei 26 %. Das Verhältnis N-(2-Propenyl)-pyrrolidin zu N-(1-Propenyl)-pyrrolidin betrug 14.

### Beispiel 10

20 g/h Ethylamin wurden in einem Vorverdampfer verdampft und dann mit 10 l/h Propin bei Normaldruck in einen Rohrreaktor bei 230°C über 100 ml des in Beispiel 9 angegebenen Hemimorphit-Katalysators umgesetzt. Der Reaktoraustrag wurde kondensiert und gaschromatographisch untersucht. Der Umsatz, bezogen auf Ethylamin, betrug 23 %. Die Selektivität für N-Ethyl-propenylamin, bezogen auf umgesetztes Ethylamin, lag bei 95 %. Das Verhältnis von N-Ethyl-2-propenylamin zu N-Ethyl-1-propenylamin betrug 12.

### Beispiel 11

20 g/h Ethylamin wurden in einem Vorverdampfer verdampft und dann mit 10 l/h Propin bei Normaldruck in einen Rohrreaktor bei 230°C über 100 ml eines amorphen Zinksilikats (die Herstellung ist beschrieben in Beispiel 7 der deutschen Patentanmeldung Nr. 19726670.3) umgesetzt. Der Reaktoraustrag wurde kondensiert und gaschromatographisch untersucht. Der Umsatz, bezogen auf Ethylamin, betrug 20 %. Die Selektivität für N-Ethyl-propenylamin, bezogen auf umgesetztes Ethylamin, lag bei 91 %. Das Verhältnis von N-Ethyl-2-propenylamin zu N-Ethyl-1-propenylamin betrug 12.

## Patentansprüche

1. Verfahren zur Herstellung von Iminen und/oder Enaminen durch Anlagerung von Ammoniak, primären Aminen oder sekundären Aminen an Acetylene und/oder Allene, dadurch gekennzeichnet, dass man die Umsetzung in der Gasphase bei erhöhter Temperatur in Gegenwart von einem amorphes und/oder kristallines Zink- und/oder Cadmiumsilikat enthaltenden Heterogenkatalysator durchführt.

2. Verfahren zur Herstellung von Iminen der Formel I und/oder Enaminen der Formel II 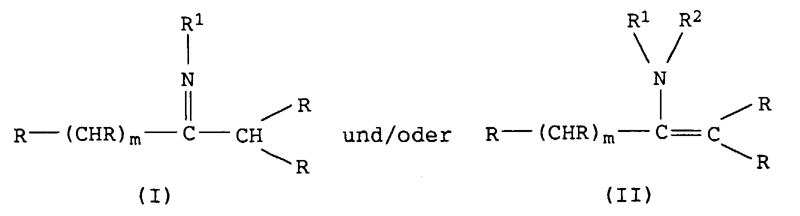 in denen die Reste R unabhängig voneinander für Wasserstoff oder aliphatische, cycloaliphatische, araliphatische, aromatische oder heterocyclische Reste stehen und R¹ und R² unabhängig voneinander Wasserstoff, aliphatische, cycloaliphatische, araliphatische, aromatische oder heterocyclische Reste bedeuten und R¹ und R² gemeinsam mit dem R¹ und R² tragenden Stickstoff einen heterocyclischen Ring bilden können und in für eine ganze Zahl von 0 bis 8 steht, durch Umsetzung von Ammoniak, primären Aminen oder sekundären Aminen der Formeln III bzw. IV
R¹NH₂ (III)
bzw. R¹R²NH (IV)
mit Acetylenen der Formel V bzw. Allenen der Formel VI 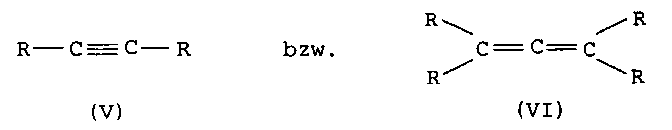 wobei R¹, R² und R die oben genannten Bedeutungen haben, dadurch gekennzeichnet, dass man die Umsetzung in der Gasphase in Gegenwart von einem amorphes und/oder kristallines Zink- oder Cadmiumsilikat enthaltenden Heterogenkatalysator bei Drücken von 0,1 bis 50 bar und Temperaturen von 100 bis 370°C durchführt.

3. Verfahren gemäß Anspruch 1, dadurch gekennzeichnet, daß man die Umsetzung in Gegenwart von Katalysatoren durchführt, die eine BET-Oberfläche von 10 bis 800 m²/g aufweisen.

4. Verfahren gemäß Anspruch 1, dadurch gekennzeichnet, daß man als Katalysator ein röntgenamorphes Zinksilikat verwendet, erhältlich durch Aufbringen eines Zinksalzes auf amorphe Kieselsäure und ggf. Formierung des Katalysators bei 50 bis 400°C.

5. Verfahren gemäß Anspruch 1, dadurch gekennzeichnet, daß man als Katalysator ein Zinksilikat mit Hemimorphit-Struktur der Formel Zn₄Si₂O₇(OH)_{2-2y}O_{y} · xH₂O verwendet, wobei x und y für die Werte 0 bis 1 stehen.

6. Verfahren gemäß Anspruch 1, dadurch gekennzeichnet, daß man als Katalysator ein bevorzugt durch Ausfällung in wässriger Lösung erhältliches im wesentlichen röntgenamorphes Zinksilikat der Formel VII
ZnₐSi_{c}O_{a+2c-0},₅ₑ(OH)ₑ · fH₂O (VII)
verwendet, in der e die Werte 0 bis zur Summe aus 2a+4c bedeutet und das verhältnis a/c 1 bis 3,5 und f/a 0 bis 200 beträgt.

7. Verfahren gemäß Anspruch 1, dadurch gekennzeichnet, daß man die Umsetzung bei Temperaturen von 150 bis 320°C und einem Druck von 0,1 bis 50 bar durchführt.

8. Verfahren gemäß Anspruch 1, dadurch gekennzeichnet, dass man primäre oder sekundäre Alkyl- oder Cycloalkylamine mit 1 bis 12 C-Atomen oder Iminogruppen enthaltende Heterocyclen mit 2 bis 8 C-Atomen und Acetylene bzw. Allene mit 2 bzw. 3 bis 8 C-Atomen verwendet.

9. Verfahren gemäß Anspruch 1, dadurch gekennzeichnet, dass man als Edukt einen Propin und/oder Allen enthaltenden Schnitt aus einem Steamcracker verwendet.

10. Verfahren zur Herstellung von Aminen, dadurch gekennzeichnet, dass man
a) in einer ersten Stufe Imine oder Enamine gemäß dem Verfahren des Anspruchs 1 herstellt und
b) die erhaltenen Imine oder Enamine unmittelbar oder nach Isolierung in an sich bekannter Weise katalytisch hydriert.

11. Verfahren zur Herstellung von Aminen durch
a) Herstellung in einer ersten Stufe von Iminen der Formel I und/oder Enaminen der Formel II 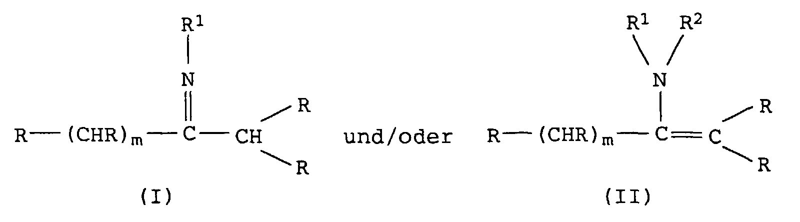 in denen die Reste R unabhängig voneinander für Wasserstoff oder aliphatische, cycloaliphatische, araliphatische, aromatische oder heterocyclische Reste stehen und R¹ und R² unabhängig voneinander aliphatische, cycloaliphatische, araliphatische, aromatische oder heterocyclische Reste bedeuten und R¹ und R² gemeinsam mit dem R¹ und R² tragenden Stickstoff einen heterocyclischen Ring bilden können und m für eine ganze Zahl von 0 bis 8 steht, durch Umsetzung von primären oder sekundären Aminen der Formeln III bzw. IV
R¹NH₂ (III)
bzw. R¹R²NH (IV)
mit Acetylenen der Formel V bzw. Allenen der Formel VI 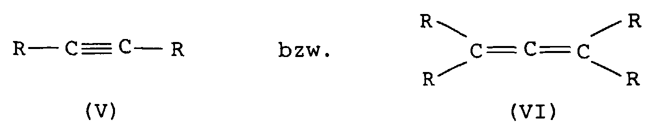 wobei R¹, R² und R die oben genannten Bedeutungen haben, und
b) katalytische Hydrierung in einer zweiten Stufe der erhaltenen Imine oder Enamine, dadurch gekennzeichnet, dass man die Umsetzung
(a) in der Gasphase in Gegenwart von einem amorphes und/oder kristallines Zink- und/oder Cadmiumsilikat enthaltenden Heterogenkatalysator bei Drücken von 0,1 bis 50 bar und Temperaturen von 150 bis 320°C durchführt und
(b) die erhaltenen Imine oder Enamine unmittelbar oder nach Abtrennung in an sich bekannter Weise katalytisch hydriert.
